# EUROPEAN PATENT APPLICATION

(11) **EP 2 108 437 A1**
(43) Date of publication of application: **14.10.2009**
(21) Application number: 08161975.1
(22) Date of filing: 07.08.2008
(51) Int. Cl.: B01D 53/85, C05F 17/02

(54) **Process and plant for purifying landfill biogas and/or removing bioaerosols through a biofiltering system, and biofilter for purifying landfill biogas and/or removing bioaerosols**

(30) Priority: 09.04.2008 IT TO20080275
(71) Applicant: Entsorga Italia S.r.l., 15057 Tortona (AL) (IT)
(72) Inventor: Cella Mazzariol, Pietro Paolo, 15057 Tortona (AL) (IT); Galanzino, Gianfrancesco, 15057 Tortona (AL) (IT)
(74) Representative: Robba, Pierpaolo

(57) **Abstract**

The present invention refers to a process and to a plant for purifying biogases generated in the landfill bodies and/or removing bioaerosols through a biofiltering system, said plant comprising at least a biofilter (1) comprising a module containing a biologically active matrix for purifying said biogases and/or removing said bioaerosols and a semi-permeable covering sheet with filtering action (10) applied onto said module.

## Description

The present invention refers to a process for purifying biogases generated in the landfill bodies and/or removing bioaerosols through a biofiltering system.

The present invention further refers to a plant comprising at least a biofilter used for purifying said biogases and/or removing said bioaerosols.

The present invention finally refers to a biofilter used for purifying said biogases and/or removing said bioaerosols.

The biogases developed in the landfills for municipal solid waste (MSW) are normally tapped by a collecting network.

At first, the produced biogas has amounts and concentrations of combustible contaminants such as to be used for generating electric power; subsequently, by decreasing the concentration thereof, the landfill biogas is oxidised at flame; in the final step, upon occurring a further production decrease, the landfill biogas is no more able to keep the flame alight and, usually, it is released to the atmosphere.

The purification of the biogases generated in the landfill bodies, and particularly of the biogases being insufficient, as to amounts and/or concentrations of combustible contaminants, to be used for generating electric power or to be removed by free flame, is a known problem in the reference technical field and it has been broadly described in literature as well.

The biogases developed in the final step, as previously said, are generally "poor" since they are characterised by a CH₄ concentration lower than 15% v/v; there are studies that consider the convenience of their removal through biofiltration before their definitive release to the atmosphere.

According to the studies carried out by Cuhls *et al.* (2002), the CH₄ degradability through biofiltration is greatly limited by lacks of oxygen or excesses of humidity.

An experimental test carried out with a biofilter filled up with foamed clay (Gebert *et al.,* 2003) highlights that the CH₄ degradation depends on temperature, with an efficiency optimum at 37 °C; the water content does not seem to be a limiting factor, while salinity and pH are to be so considered.

Streese *et al.* (2003) suggest a sizing for biofilters serving landfills much more higher than that defined for the smells treatment, the flow being equal. In their work, by pointing out the inadequacy of the conventional biofilters consisting of wood splinters and compost, they tested an equally divided mixture of wood splinters, compost and peat as well as a stratified arrangement of compost layers with wood splinters layers placed therebetween; in particular, the mixture was tested both at laboratory and in a closed landfill, by mixing biogas and ambient air until obtaining a CH₄ content of 0.2 - 2.5% and with a biofilter sizing of about 5 m³/(h/m³). The results confirm the inefficiency of the peat and indicate that the removal efficiency by the compost, up to about 70 g/(m³/h) depending on the CH₄ concentration, is higher than both the mixture wood/compost/peat and their stratified arrangement compost/wood. Moreover, the mixture wood/compost/peat proves to be the best material on the long period (up to 350 days) thanks to a smaller sensibility towards the extra-polymeric cellular substances (EPS), which consist of methanotrophic bacteria and which tend to clog up the substrate. The study also highlights that the compost biofilter has an oxidation potential higher than that of the mixed material biofilter but it has more inefficient mass transfer, the performance of the latter therefore resulting the better in all. Among the problems raised during said study, one of the more significant is the filtering matrixes desiccation, due to the heating following the CH₄ oxidation (up to about 50 °C) not counterbalanced by high air flows.

The field's technical literature also cites the possibility of using biofilters operating with passive ventilation generated by the pressure gradient between the landfill body and the external environment; however, this solution shows some disadvantages linked to the flow irregularity (Straka *et al.,* 1999; Gebert *et al.,* 2001) and to the oxygen withdrawal from the surface in counter-current towards the biogas flow.

There still exists, therefore, the need of finding a solution for the biological treatment of the "poor" biogases extracted from landfills that overcomes the disadvantages above described and that takes into consideration the filtering matrix kind, the need of diluting the biogas with ambient air, the biofilter sizing, the influence of the temperature, the CH₄ explosivity, the EPS formation and the possible problems caused by other contaminants, such as H₂S.

Biofiltration is anyhow a technology known and commonly used mainly for deodorising the emissions from waste treatment plants.

The operating principle of a biofilter provides for using a wide spectrum of chemoautotrophic and chemoheterotrophic micro-organisms (such as, for instance, bacteria, actinomycetes and fungi) that, by colonising the filtrating matrix, are able to metabolise the substances present in the gaseous effluents passing therethrough, if they are either natural or synthesis, either inorganic or organic, either aliphatic or aromatic compounds.

The biofilters efficiency in removing smells and potentially toxic substances is high, and such as to effectively maintain the smelling emissions below the law thresholds; however, a worry raised in their employ concerns the potential emission from the filtering surface of the so-called bioaerosols, that is of vapours and powders containing moulds, bacteria and other organisms potentially bad, at high concentration, for the health of people working near the plants.

Among the found mycetes species, the spores of *Aspergillus fumigatus* result to be particularly worrying; the *Aspergillus fumigatus* is a non-pathogen mould that however, in case of prolonged and massive inhalation, can support unhealthy forms with proliferation at pulmonary level (aspergillosis) and that has an optimum increase temperature of about 37 °C.

According to some analysis carried out on biofilters in the active phase, that is fed with hot air coming from municipal solid waste (MSW) treatment plants, the presence of fungal spores is generally lower than 1,000 Colony-Forming Units (CFU)/Nm³, comparable to those normally existing in rural environments; however, it has been recently highlighted the possibility that the emissions from biofilter exceed 10,000 CFU/Nm³, with presences of *Aspergillus fumigatus* significant in percentage.

Even specifying that, at present, no regulations exist in this respect, the indications at European level place the acceptable risk contents at about 1,000 CFU/Nm³ for the overall fungi and bacteria; some studies, on the contrary, deem that concentrations of *Aspergillus fumigatus* spores below 2,000 CFU/Nm³ are acceptable.

There still exists, therefore, also the need of finding a solution for the biological treatment of the bioaerosols that overcomes the disadvantages above described.

The Applicant has found (i) a process for purifying biogases generated in the landfill bodies and/or removing bioaerosols through a biofiltering system, (ii) a plant comprising at least a biofilter used for purifying said biogases and/or removing said bioaerosols, and (iii) a biofilter used for purifying said biogases and/or removing said bioaerosols, that overcome the disadvantages above described thanks to the provision of a semi-permeable covering sheet with filtering action located onto said at least a biofilter.

A preferred embodiment of the plant comprising at least a biofilter as well as of the biofilter according to the invention will be now described with reference to the following figures, given by way of non limiting example, in which:
- FIG. 1 is a schematic representation of the biogases and/or bioaerosols treatment plant comprising at least a biofilter according to the invention;
- FIG. 2 is a perspective view of a biofilter, without the semi-permeable covering sheet, according to the invention;
- FIG. 3 is a perspective view of the biogases and/or bioaerosols treatment plant showing the feed system of the biogases to the biofilters according to the invention;
- FIG. 4 is a perspective view of the plant of FIG. 3 showing the biofilters, equipped with the semi-permeable covering sheet, according to the invention;
- FIG. 5 is a schematic representation of the lay-out of the biogases and/or bioaerosols treatment test plant according to the invention; and
- FIGG. 6a, 6b, 6c and 6d are graphics representing the evolution of the temperature profiles detected at two different depths in the biofiltering matrix in the first, in the second, in the third and in the fourth biofilter, respectively, of the biofiltering system in the test plant.

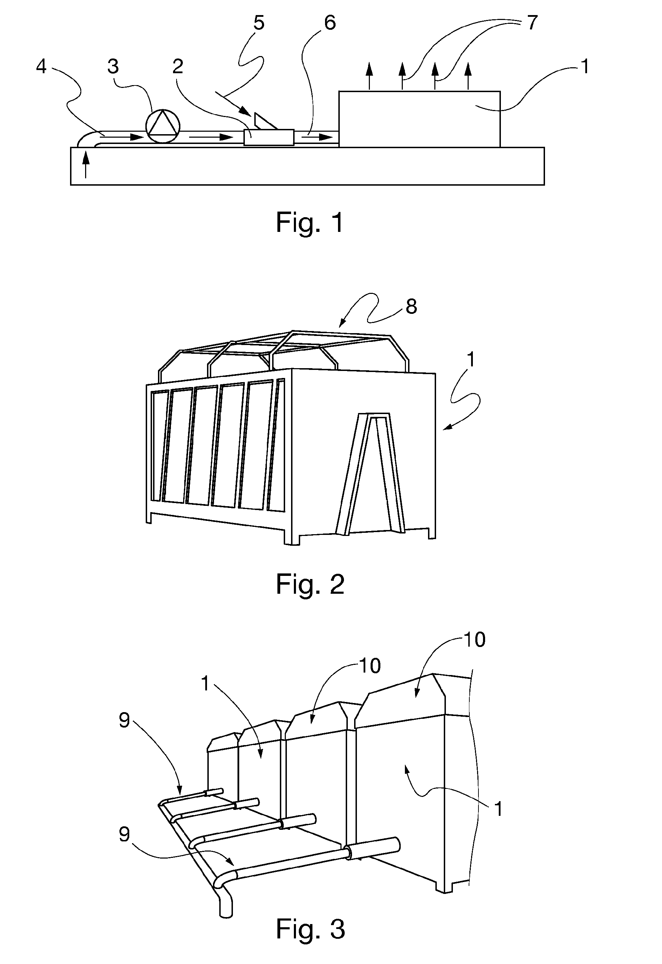

With reference to FIG. 1, a biogases and/or bioaerosols treatment plant is visible, comprising at least a biofilter 1 according to the invention.

Said at least a biofilter 1 preferably consists of a roll-off biofiltering module, as shown in FIG. 2.

Preferably, said roll-off biofiltering module is made of steel, more preferably of COR-TEN steel; preferably said module is 6.5 m x 2.5 m x 2.7 m sized, equal to an internal useful volume of about 25 m³.

Said biofilter 1 comprises a settling chamber, or plenum, (not shown) positioned on its lower portion; inside said chamber the gas to be purified 4 is blown in, preferably through side entrances 9, as shown in FIG. 3.

Going back to FIG. 1, it is noticed that, depending on the application needs, the plant comprising the at least a biofilter 1 can be provided with a predilution device, or mixer, 2 of the biogas to be treated 4 with ambient air 5, in order to produce a mixture 6 wherein the inlet methane concentration to said biofilter 1 is approximately within the threshold of 3%; said biogases 4 are fed to said mixer 2 preferably through a fan 3, and said biogas/air mixture 6 is then sent to the biofilter 1, which carries out the purification thereof and from which the purified air 7 is discharged.

The biofiltering matrix (not shown) is laid down over the plenum and separated therefrom by means of a holed paving (not shown).

Said biofiltering matrix preferably consists of a preconstituted mixture, comprising preactivated ligno-cellulosic oversize material coming from composting plants, compost and peat.

Said biofiltering matrix preferably occupies a volume of about 22 m³, this involving a treatment capacity of about 100 m³/h of said biogas/air mixture 6 within the methane concentration threshold above specified.

Said biofiltering matrix can be periodically humidified, for instance with water preferably coming from a system of nozzles 8, preferably having a temporised opening, installed over the surface of said biofiltering matrix, as shown in FIG. 2; the wetting frequency and intensity can be established and modified depending on the specific application needs.

The activity of the biofiltering material can be monitored through the measurement of parameters such as the temperature and the relative humidity of the biogas at entrance and of the biofiltering matrix, the concentration of the methane at entrance into the system and upon exit from the biofiltering matrix, the flow of the biogas and of the mixing air.

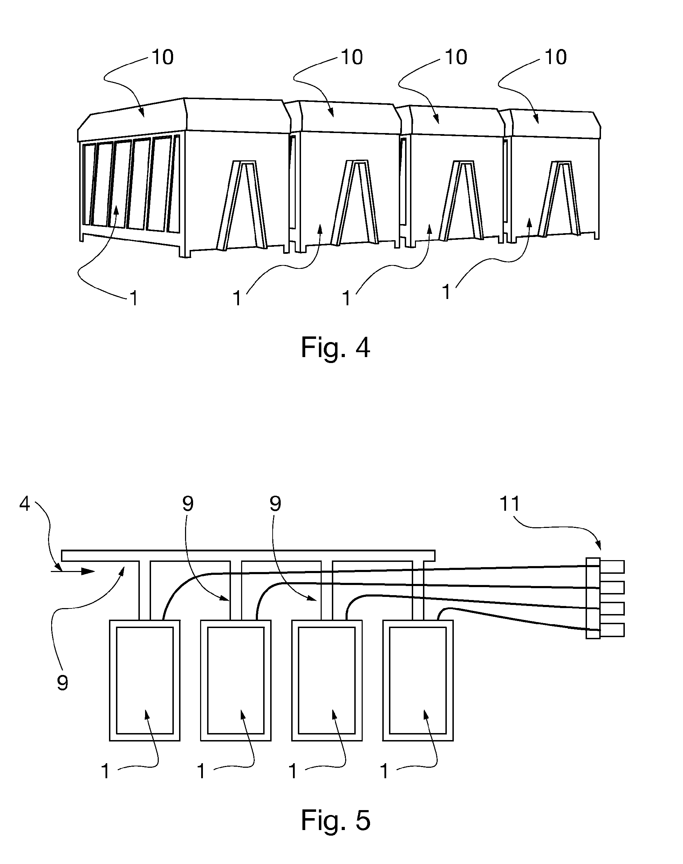

Making now reference to FIG. 4, it is noticed, in particular, a battery of biofilters 1 loaded up with the mixtures of biologically active matrixes previously described and to which a semi-permeable covering sheet with filtering action 10 is applied, over said matrix.

Preferably, said sheet 10 is located over said system of nozzles 8.

Said sheet 10 is made of a synthetic fabric having high impermeable and transpiring abilities; preferably said synthetic fabric consists of thermo-mechanically foamed polytetrafluoroethylene (PTFE).

Said sheet has about 9 billions of microscopic holes for square inch; each hole is about 20,000 times smaller than a water drop, but it allows the aqueous vapour produced by the evaporation to pass through, making the fabric transpiring.

The Applicant has surprisingly found that the employ of said sheet 10 allows to carry out a further filtration of the biogases, this causing an overall improvement of the plant efficiency.

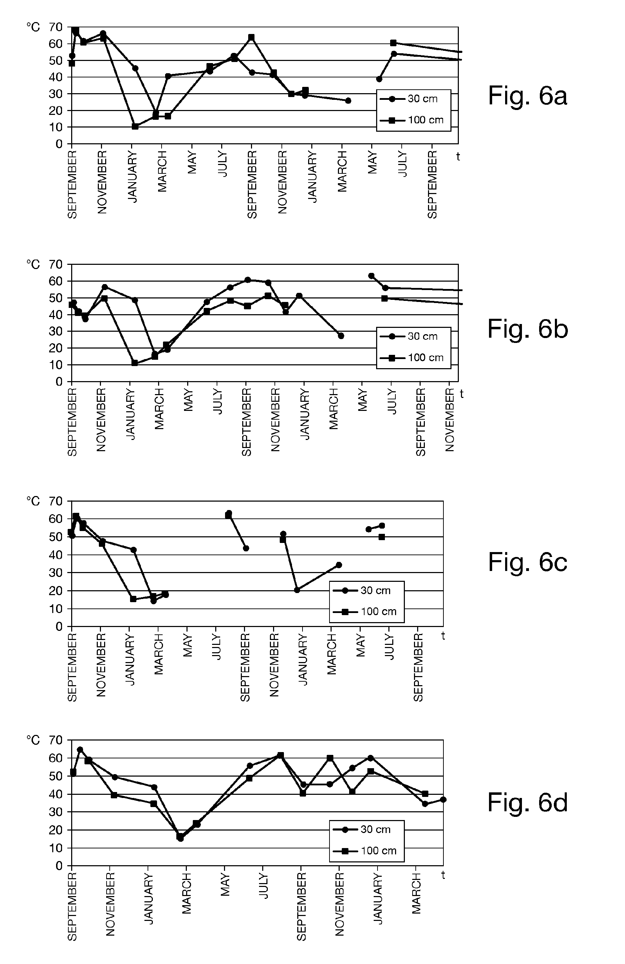

The Applicant has experimentally tested the validity of the present invention, experimental activity that will be now illustrated with reference to FIGG. 5 and 6.

The performed experimental activities provided, besides the maintenance activities suitable for maintaining the optimum working conditions of the biofilters, also the monitoring of the main operating parameters, such as flows and temperature of the biogas, temperatures and microbic colonisation status of the biofiltering matrix, methane concentration upstream and downstream the biofiltering system.

The test plant, shown in FIG. 5, included a battery of four biofilters 1 according to the present invention, loaded up with convenient mixtures of biologically active matrixes, a system of feed lines of the biogas 9 and a detecting/monitoring/adjusting system 11 of the main operative parameters connected to said biofilters 1.

The biogas conveyed to the test plant had a methane concentration on average lower than 15% v/v and an average overall flow of about 400 m³/h.

During the first phase of the experimental activity three biofilters have been left uncovered while a semi-permeable covering sheet with filtering action has been applied onto the fourth one; such covering sheet was applied also onto the first three biofilters during the second phase of the experimental activity, this allowing to increase the biogas flows fed to each biofilter.

During the experimental test, the continuous monitoring of the plant operative conditions highlighted that the methane average percentage upon exit from the plant consolidated at values comprised in the range 6% - 9% v/v, with an average decrease of 9% - 6% v/v compared with the content at entrance into the plant.

The check of the right functioning of the biofiltering system, besides the estimate of the methane removal efficiency, has been performed by means of periodical measurements of the temperatures of the biofilters, precisely of the biofiltering matrixes, at two different depths, of 30 cm and 100 cm respectively; the evolution of the temperature profiles detected at said two different depths, in the first, in the second, in the third and in the fourth biofilter, respectively, of the biofiltering system in the test plant, is shown in FIGG. 6a, 6b, 6c and 6d.

All the biofilters forming the test plant highlight similar temperatures trends, decreasing in the winter period and recovering starting from the month of March; in particular, it has been noticed that the presence of the semi-permeable covering sheet caused, during the winter months, the maintenance of on average high temperatures, thanks to the better conservation of the humidity as well as of the heat exiting from the biofiltering matrix.

The temperatures detected at 30 cm of depth have always shown values higher than those detected at 100 cm of depth; it is deemed, without intending to be bound by this hypothesis, that a greater microbic activity exists in the upper portion of the biofiltering matrix, due to the contribution of the ambient air in the exchange of gases, such as O₂ and CO₂, with the biologically active material.

Moreover, the check of the right functioning of the biofiltering system has been performed by means of periodical measurements of the microbic colonisation status of the matrixes; more precisely, both the quantitative evolution of the microbic consortium expressed as "total bacterial load" and the quantitative evolution of the mycetic consortium expressed as "total mycetic load" have been determined.

As far as the microbic colonisation is concerned, from a point of view of both the bacterial load and the mycetic load, no poisoning symptoms of the biofiltering matrix due to the biogas subjected to treatment have been found; the evolution of the consistence of the microbic populations seems to indicate a selection in favour of the bacterial component, to which the methanotrophic micro-organisms belong.

The detected data have also proved the validity of the application of the semi-permeable sheet while, on the contrary, using a totally impermeable sheet would have brought to the extinction of the microbic colonies in the biologically active matrix.

Finally, analyses on the leachate produced by the biofilters of the test plant have been carried out, which showed a very low presence of polluting substances.

On the basis of the performed measurements, a relation could be recognised between the biogas flows fed to the biofilters and the degradation capacity of the methane contained in the biogas; from said measurements, it appears the direct correlation between the biogas flows fed to the biofiltering system and the degraded methane and the removal efficiency respectively.

Furthermore, by considering the average results relevant to the four biofilters of the test plant, a slight decrease of the methane removal efficiency is observed when passing from lower biogas flows to higher ones, as it results from the below table:

| | **FLOW** | **EFFICIENCY** |
|---|---|---|
| | **[m³/h]** | **[%]** |
| **BIOFILTER 1** | 123.7 | 71.8 |
| **BIOFILTER 2** | 128.7 | 68.5 |
| **BIOFILTER 3** | 93.2 | 68.6 |
| **BIOFILTER 4** | **51.7** | **80.4** |

From the test carried out by the Applicant it results, moreover, that the combination of biofiltering matrix and semi-permeable covering sheet allows to achieve high removals of the biogas smelling impact; the olfactometric surveys carried out according to UNI EN 13725-2004 regulation, have in fact highlighted excellent removal levels, generally higher than 90% of the olfactometric units at entrance, and values lower than the threshold limit commonly considered to be of 300 OU/Nm³.

To conclude, it has been found that the application of the semi-permeable covering sheet with filtering action performs an essential purifying action, which acts by improving the removal of both the methane and the smells.

Such surprising result is confirmed by the microbiological analyses carried out on the aqueous film that is formed in the intrados of the semi-permeable sheet applied onto the biofilter, reported in the below table, from which it is deduced a significant presence of active microbic populations:

| **Sample** | **Parameter** | **Result** |
|---|---|---|
| Semi-permeable sheet buffer | Total bacterial load | 500 CFU/cm² |
| | Total mycetic load | 1 CFU/cm² |

The Applicant believes that the formation of said aqueous film, consisting of water droplets containing active microbic colonies, significantly contributes to the improvement of the biofilter efficiency in terms of biogas purification, since inside said film an additional filtration would occur.

Within the test carried out by the Applicant on the biofilters covered with semi-permeable covering sheets with filtering action, as described above, the possibility of achieving also the removal of bioaerosols is raised, since the sizes of the bioaerosols spores, and in particular the sizes of the *Aspergillus fumigatus* spores, are larger than the micro-holes of the transpiring membrane used, and this latest acts as a sieve for the polluting substances considerably reducing the emission thereof.

In this way, the applied semi-permeable covering sheet with filtering action according to the invention performs a double action: the further removal of the smells, but overall the removal of the bioaerosols, and the overall removal efficiency of the biofilter passes from 80% to more than 99%.

As far as the process for purifying biogases and/or removing bioaerosols according to the invention is concerned, it comprises the following steps:
- arranging for at least a biofilter 1 having the features above described;
- positioning a semi-permeable covering sheet with filtering action 10 onto said at least a biofilter 1;
- arranging for a detecting/monitoring/adjusting system 11 of the main operative parameters connected to said at least a biofilter 1;
- feeding the biogases 4 to said at least a biofilter 1, preferably through a fan 3;
- allowing said biogases 4 to be purified and/or said bioaerosols to be removed inside said at least a biofilter 1; and
- allowing the purified air 7 to be discharged from said at least a biofilter 1.

Optionally, said process for purifying biogases and/or removing bioaerosols further comprises the following step:
- mixing the biogas to be treated 4 with ambient air 5 inside a mixer 3, in order to produce a biogas/air mixture 6, which is then sent to the biofilter 1.

Optionally, said process for purifying biogases and/or removing bioaerosols further comprises the following step:
- periodically spraying said at least a biofilter 1, and precisely the biologically active matrix therein contained, with water preferably coming from a system of nozzles 8, preferably having a temporised opening, installed over the surface of said matrix.

Optionally, said process for purifying biogases and/or removing bioaerosols further comprises the following step:
- allowing the formation of an aqueous film containing active microbic colonies in the intrados of said semi-permeable covering sheet with filtering action 10 of said biofilter 1.

From the description above depicted in detail, the advantages ensuing the process for purifying biogases generated in the landfill bodies and/or removing bioaerosols through a biofiltering system, the plant comprising at least a biofilter used for purifying said biogases and/or removing said bioaerosols and the biofilter used for purifying said biogases and/or removing said bioaerosols according to the present invention result clear; in particular:
- the drastic decrease of the methane compounds released to the atmosphere that, as known, have an effect on the climate change of good 20 times higher than that of CO₂, the amount being equal; in that sense, the present invention significantly cooperate to the fulfilment of the Kyoto Protocol;
- the possibility to operate in a safe and efficient way the purification of biogases coming from landfills, thus reducing the overall environmental impact thereof;
- the mobility and the modularity of the biofiltering system, which allows the sizing thereof for meeting the treatment actual needs, the positioning thereof near the biogases tap system and the easy adjustment thereof to the specific application;
- the limited investment costs;
- the contained operational and maintenance costs;
- the building simplicity and compactness;
- the maintenance simplicity; and
- the continuous and reliable functioning.

It is clear that the process for purifying biogases generated in the landfill bodies and/or removing bioaerosols through a biofiltering system, the plant comprising at least a biofilter used for purifying said biogases and/or removing said bioaerosols and the biofilter used for purifying said biogases and/or removing said bioaerosols according to the present invention, herein described by means of a preferred embodiment, given by way of non limiting example, can be modified according to ways known to the person skilled in the technical field without falling out the scope of the present invention.

## Claims

1. A biofilter (1) comprising:
- a module containing a biologically active matrix; and
- a semi-permeable covering sheet with filtering action (10) applied onto said module.

2. A biofilter according to claim 1, wherein said biofilter (1) further comprises a system of nozzles (8) installed over the surface of said biologically active matrix to periodically humidify said surface with water coming from said nozzles (8), said nozzles (8) having a temporised opening.

3. A biofilter according to claim 2, wherein said semi-permeable covering sheet with filtering action (10) is located over said system of nozzles (8).

4. A biofilter according to claim 1, wherein said semi-permeable covering sheet with filtering action (10) is made of a synthetic fabric having high impermeable and transpiring abilities, said synthetic fabric consisting of thermo-mechanically foamed polytetrafluoroethylene (PTFE) and having about 9 billions of microscopic holes for square inch, each hole being about 20,000 times smaller than a water drop.

5. A biofilter according to claim 1, wherein said semi-permeable covering sheet with filtering action (10) comprises, in its intrados, an aqueous film containing active microbic colonies.

6. A biofilter according to claim 1, wherein said module is a roll-off device.

7. A biofilter according to claim 1, wherein said biologically active matrix consists of a preconstituted mixture, comprising preactivated ligno-cellulosic oversize material coming from composting plants, compost and peat.

8. A plant for purifying biogases and/or removing bioaerosols comprising at least a biofilter (1) according to one of the claims 1 to 7.

9. A plant according to claim 8, further comprising a fan (3) for feeding the biogases to be treated (4), said feeding of the biogases to be treated occurring through side entrances (9).

10. A plant according to claim 8 or 9, further comprising at least a predilution device, or mixer, (2) of the biogas to be treated (4) with ambient air (5).

11. A process for purifying biogases and/or removing bioaerosols comprising the steps of:
- arranging for at least a biofilter (1) according to one of the claims 1 to 7;
- positioning a semi-permeable covering sheet with filtering action (10) onto said at least a biofilter (1);
- arranging for a detecting/monitoring/adjusting system (11) of the main operative parameters connected to said at least a biofilter (1);
- feeding the biogases (4) to said at least a biofilter (1);
- allowing said biogases (4) to be purified inside said at least a biofilter (1); and
- allowing the purified air (7) to be discharged from said at least a biofilter (1).

12. A process according to claim 11, wherein the feeding of the biogases (4) to said at least a biofilter (1) occurs through a fan (3).

13. A process according to claim 11 or 12, further comprising the steps of:
- mixing the biogas to be treated (4) with ambient air (5) inside a mixer (3), in order to produce a biogas/air mixture (6), which is then sent to the biofilter (1);
- periodically spraying with water said at least a biofilter (1), and precisely the biologically active matrix therein contained.

14. A process according to claim 13, wherein the spraying water comes from a system of nozzles (8) installed over the surface of said matrix, said nozzles (8) system being a temporised opening system.

15. A process according to claim 11, 12, 13 or 14, further comprising the step of:
- allowing the formation of an aqueous film containing active microbic colonies in the intrados of said sheet (10) of said at least a biofilter (1).
